# EUROPEAN PATENT APPLICATION

(11) **EP 1 439 153 A1**
(43) Date of publication of application: **21.07.2004**
(21) Application number: 04250137.9
(22) Date of filing: 13.01.2004
(51) Int. Cl.: C04B 12/02, A61L 24/02, A61L 24/00, A61K 6/06

(54) **Calcium phosphate cement precursors**

(30) Priority: 17.01.2003 US 346873
(71) Applicant: Howmedica Osteonics Corp., Mahwah, NJ 07430 (US)
(72) Inventor: Zitelli, Joseph, River Edge, New Jersey 07661 (US); Mahwer, Peter, Patrickswell, Co. Limerick (IE); Insley, Ger, Rathkeale, Co. Limerick (IE)
(74) Representative: Green, Mark Charles

(57) **Abstract**

A mixture of two or more calcium phosphate compositions are combined with an aqueous solution and are useful as bone fillers on bone cements such as orthopedic and dental cements and remineralizers. The mixtures self-harden to substantially form hydroxyapatite, the mineral phase of bone and tooth enamel. The at least one or more of precursors is prepared in a manner resulting in its having an extremely low moisture level. The use of low moisture components results in an improved shelf life to the mixture.

## Description

### BACKGROUND OF THE INVENTION

This invention relates to calcium phosphate compositions, including cement and pastes for orthopedic applications and to methods for making and using them. In particularly, it relates to improved storage properties and shelf life for hydroxyapatite (HAP) forming calcium phosphate precursors particularly those including dicalcium phosphate.

The microstructural features of teeth and bone and the process by which remodeling occurs are of importance in determining desirable microstructures in bone-like substitutes. Because bone substitutes must emulate the function of bone, bone must bond well with them.

U.S. Patent Nos. 4,880,610, 5,047,031 and 5,053,212 to Constantz, et al. and assigned to Norian Corporation disclose calcium phosphate compositions useful as bone cements. The process for forming these cements involves merely mixing two dry ingredients, i.e., a dry calcium source and dry phosphoric acid with aqueous solution to form a kneadable mixture.

In U.S. Patent No. 5,053,212, water is liberated in the mechanical mixing of the calcium source with the phosphoric acid. Disadvantageously, this liberation of water is free to combine with the calcium and phosphoric acid which results in calcium phosphates which are slow to react to form HAp, and upon reaction to form HAp again release water, thereby increasing the porosity and limiting the mechanical strength of the resultant product.

U.S. Patent No. 5,496,339 assigned to Norian Corporation relates to a storage stable calcium pholsphate cement in which the two component apatitic cement composition has two components which are acid and base sources premixed as a single dry ingredient to be used with an aqueous solution to form a setting cement. In order to prevent any reaction between the stored two components neutralization of the dry acidic phosphate source is stopped prior to completion through the removal of available water. One way of stopping the reaction between the acid and the base is by removing the water using a freeze-drying technique. Usually this reaction will be stopped at a point of less than about 50% of completion. Such a process requires the precise control and the close monitoring of the reaction.

Brown and Chow U.S. Reissue Patent Nos. 33,161 and 33,221, the teachings of which are incorporated herein by reference, and the Brown and Chow publication "A New Calcium Phosphate, Water Setting Cement" Cement Research Progress (1986) disclose mechanically mixing dry tetracalcium phosphate Ca₄(PO₄)₂O (TTCP) with at least one other dry calcium phosphate selected from the group consisting of dicalcium phosphate dihydrate CaHPO₄· 2H₂O, dicalcium phosphate anhydrous CaHPO₄, octacalcium phosphate Ca₈H₂ (PO₄)₆· 5H₂O, (i.e. Ca₈ (HPO₄)₂(PO₄)₄· 5H₂O), tri-calcium phosphate α-Ca₃ (PO₄)₂, β-Ca₃(PO₄)₂ and modified tri-calcium phosphate Ca₃(PO₄)₂ for use in a dental restorative powder, paste and/or slurry. While the mechanical mixing of the two dry components does not cause a reaction between the TTCP and the at least one other calcium phosphate, storing the mixture under humid conditions for long periods of time can cause some deterioration in the precursors.

The above discussed patents to Brown and Chow and Constantz, et al. rely on grinding mixtures of dry particles to a high fineness, which particles in admixture are then reacted in an aqueous solution to form hydroxyapatite.

As set forth above, one composition detailed by Brown and Chow is a combination of powdered tetracalcium phosphate (TTCP) and either powdered dicalcium phosphate anhydrous (DCPA) or dicalcium phosphate dihydrate (DCPD). These sparingly soluble calcium phosphates when combined in an aqueous solution fuel a cementitious reaction to form hydroxyapatite Ca₁₀(PO₄)₆OH. Chow and Takagi U.S. Patent Nos. 5,522,893, 5,542,973 and 5,695,729, the teachings of which are hereby incorporated by reference, teach the use of a specially processed TTCP as one of the reactants. The TTCP is made with a calcium to phosphorous ratio of less than 2 (its stoichiometric value) and it is prepared and maintained anhydrously. These patents state that calcium phosphate cements with substantially improved setting times and mechanical strengths are obtained when the tetracalcium phosphate is prepared under anhydrous conditions. The resulting cements set reliably with concomitant improved strength attainment. The setting rate can be adjusted for various end uses, and may be quite rapid if desired. For example, sodium phosphate can be added to the aqueous component (deionized water, for example) to speed the reaction. The resulting hydroxyapatite cement is both biocompatible and resorbable (biodegradable) with bone replacement when in contact with living bone. Thus, the resulting combination of this TTCP powder with DCPA and/or DCPD powder has improved properties such as more rapid and more reliable set times when mixed with an aqueous setting solution.

The precursor materials of Brown and Chow, Chow and Takagi, while useful in their current configuration when freshly made, are susceptible to a deterioration of some of their properties over time when stored as a dry mixture. As described by Chow and Takagi in U.S. Patent No. 5,522,893, if TTCP is exposed to moisture the strength properties of the resulting cements decrease with increased exposure to 100% humidity moist air.

### SUMMARY OF THE INVENTION

It has now been discovered that processing of the other sparingly soluble calcium phosphate used in combination with the TTCP, can improve the long term storage properties of the cement powder composition. Specifically, it is known that if the DCPA (which is commercially available from J.T. Baker) is the sparingly soluble calcium phosphate precursor used in combination with TTCP it needs to have a specific particle size to be useful as a component in the cement. It has been found that this size is typically in an approximate 1:10 particle size relationship with TTCP. The TTCP has a typical size of between about 10-20 microns (µm) and usually about 14 or 15 µm.

The commercially available DCPA has a particle size of about 14-15 µm and needs to be reduced in particle size preferably to about 1-2 µm to be used with dry TTCP as a dry precursor to the cement. It should be noted that the particle size measurements were all done by the same method as using different methods may result in inconsistent results. This particle size is difficult to attain with dry milling methods. Consequently, non-aqueous wet milling techniques have been tried in an attempt to reduce residual moisture levels. This results in difficulty in removing both the non-aqueous and miscible aqueous components of the ground wet slurry.

Wet aqueous milling methods have been used with standard drying techniques such as oven drying and vacuum drying but these techniques have the potential to cause residual moisture to be returned in the DCPA which can contaminate the TTCP particles during storage with the DCPA as a mixture. It is believed that this residual moisture has the same effect on TTCP over time during storage as does exposure to moist air. In order to test this theory, an accelerated aging test (sealing the TTCP-DCPA mixture in a glass container and placing it in a 60°C oven for eight (8) days) has been developed. This test shows whether or not the residual moisture has had an effect on the TTCP and degraded the cementitious reaction. Alternately, a standard aging test of letting the powder combination sit in an airtight container for about six (6) months has also been used to test the effect of shelf life on the setting time of the cement. The six months was chosen since this is the minimum acceptable shelf life and longer tests could be performed if desired.

### DETAILED DESCRIPTION

Surprisingly, it has been found that wet aqueously ground DCPA can be successfully used in a TTCP-DCPA cement and setting times controlled if certain post grinding drying procedures are followed. It has been found that the freeze drying of the resulting water based DCPA slurry constantly produces an extremely dry finely ground DCPA powder, which is especially suitable for combination with anhydrously produced TTCP (per the process of U.S. Patent Nos. 5,522,893, 5,542,973 and 5,695,729). This combination of TTCP and DCPA was found to be especially stable in accelerated aging and standard aging tests. Freeze drying of an aqueous slurry of fine particles can produce very dry particles. These are especially well suited for long term storage as a powdered mixture with a material such as TTCP which can have its properties degraded when exposed to moisture.

In the preferred milling operation, DCPA powder of 14-15 µm particle size are mixed with deionized water to form thin slurry with a solids concentration of about 33% by weight. It is pumped into a 600 ml bead mill with 0.6 to 0.8 mm zirconia balls at a rate of under 16 ml/min. Such a mill may be the Dyno-Mill IM Model KDL (manufactured by WA Bachofen AG in Switzerland). The discs moving the zirconia balls are preferably rotating at about 10 m/sec or higher and more preferably at 14.0 m/sec or about. These are two of the speeds indicated as choices on the Dyno-Mill KDL. The slurry which exits the bead mill is a thick paste, which indicates the DCPA particles have been made much finer with an increased surface area. The resulting particle sizes are generally in the range of preferably about 1-2 µm.

In the preferred method this slurry is made into a film of 15 mm or less in thickness and the film is frozen preferably by immersion in liquid nitrogen or by other suitable means, to a low temperature. The moisture is removed from the frozen slurry by sublimation due to exposure to a vacuum.

In the preferred embodiment after freezing the slurry and while still frozen, the film is dried to remove the entrained liquid. This procedure may be accomplished by placing the frozen slurry into a vacuum chamber for a given period of time. Preferably, this is about 24 hours. Alternately, the slurry may be placed in a container which is then connected to a vacuum. Freeze-drying generally occurs at liquid nitrogen temperatures and for a time in the range of about 12-78 hours preferably about 24 hours and under a vacuum range of about 10⁻³ to 10⁻⁵ torr. A preferred method includes lyophilization because the liquid nitrogenic temperatures typically used in lyophilization inhibit further crystallization of the calcium phosphate material. Such further crystallization is undesirable.

This freeze drying process results in very low residual moisture levels, and a light fluffy powder of ground DCPA. This powder has an average particle size of 1-2 µm. This powder is particularly well suited for combination with anhydrous TTCP for use as the powdered component in forming a calcium phosphate cement. Once mixed, the powders are sealed in an airtight or moisture proof container and sterilized by conventional methods.

### EXAMPLE I

500 grams of DCPA powder mean particle size 14 µM was obtained from J.T. Baker (Lot #34614). This powder was combined with 1000 ml of deionized water to produce a slurry and kept in suspension with a stir bar on a magnetic stirrer. A peristaltic pump was used to feed the slurry into a Dyna-Mill KDL 600 ml bead mill. The 600 ml glass grinding chamber was charged with 500 ml of 0.4 mm to 0.6 mm zirconia balls (beads). The grinding balls were driven by a series of discs with a speed of about 14.0 m/sec. The first 600 ml of slurry pumped through the mill was discarded. Thereafter, a thick slurry was being produced by the mill at about 16 ml/min. This material was retained for the freeze drying process.

A small amount of slurry was placed into a 40 ml glass vial and swirled to coat the inner glass surface. This vial was then plunged into liquid nitrogen held in an insulated container. After the contents were frozen, the vial was sealed and connected to the vacuum port on a Virtis 3+SL freezer dryer. A vacuum of 10⁻⁴ torr was applied. It remained there for about 24 hours or until all the moisture was removed as shown by the precursors of a free flowing powder in the glass vial.

This powder was the mixed in a 1:1 molar ratio with anhydrous TTCP made according to the process of Chow and Takagi U.S. Patent No. 5,522,893 to produce a calcium phosphate cement forming powder.

When the DCPA made by the above-described process and the TTCP made by the process of the 5,522,893 patent were mixed with deionized water at a 0.30 L:P (liquid/powder) ratio the cement set (or self hardened) in 5 minutes. Set time was determined by mixing the paste on a glass slab and placing it in a 3 mm thick steel mold with a 6 mm hole in the center. After the paste was in the mold it was sealed with plastic cover slips and clamped tightly shut. It then was placed in a 37°C humidity cabinet at 100% humidity until the set tests are performed at various time intervals. The paste is considered set if a 454 gm 1.06 mm diameter gilmore needle will not make an indentation in the surface. The gilmore needle is slowly (12.5 inches/min) dropped on the surface of the setting cement. Multiple set tests were done to determine at which time point the needle will no longer penetrate the cement paste. This resulted in a setting time of 5 minutes.

This same TTCP and DCPA powder mixture was placed in a glass jar and sealed with a rubber stopper and aluminum crimp top. The glass jar was placed in a 60°C oven for 8 days. When retested with the same set testing procedures as above, the cement again has a 5 minute set time. This test shows that there is no degradation in set times even after accelerated aqing of the TTCP/DCPA powder mixture in a package.

### Example II

Fine DCPA made according to Example I and anhydrous TTCP made according to Chow and Takagi U.S. Patent No. 5,522,893 were combined in a 1:1 molar ratio. Using a 0.3 liquid to powder ratio, with deionized water, a paste was made from the powder mixture and tested for set times as in Example I. The cement paste sets in 5 minutes. The same powder was placed in an air-tight glass jar and sealed according to Example I. The jar remains in a standard laboratory room environment for 176 days. When tested for set as in Example I in this non-accelerated (real-time) aging environment, the cement paste set in 5 minutes. Consequently, with regard to set time the long term storage properties of the TTCP powder/DCPA powder combination of the present invention are very stable.

While it is preferred that the milled material be entirely DCPA, small amounts of DCPD do not materially effect the setting times. It has been found that milling at high temperatures (40-50° C) reduces the amount of DCPD in the slurry produced by the mill. In addition, while DCPA is the preferred ingredient with TTCP, it is also possible to use tri-calcium phosphate and octacalcium phosphate in the milling process.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims. For example, although decalcium phosphate anhydrous was used any other sparingly soluble calcium phosphate such as those taught in the Norian, Brown and Chow patents could be utilized.

## Claims

1. A method for producing a powdered precursor for a calcium phosphate cement comprising:
milling a dicalcium phosphate powder slurry using a wet milling method;
freeze-drying the dicalcium phosphate after processing by the wet method to produce a dry powder;
mixing the freeze-dried dicalcium phosphate powder with a second powdered component; and
packaging the mixture in a moisture proof container.

2. The method as set forth in claim 1 wherein said dicalcium phosphate powder has a particle size of about ten times greater before said milling.

3. The method as set forth in claim 2 wherein the dicalcium phosphate has a particle size of between about 1-2 µm after milling.

4. The method as set forth in claim 3 wherein the second material is tetra-calcium phosphate (TTCP).

5. The method as set forth in claim 4 wherein the TTCP has a particle size of between about 10-20 µm.

6. The method as set forth in claim 1 wherein the second powdered component is tetracalcium phosphate.

7. The method as set forth in claim 6 wherein the tetracalcium phosphate is made with a calcium to phosphorous ratio of less than 2.

8. The method as set forth in claim 7 wherein the tetracalcium phosphate is prepared and maintained anhydrously.

9. The method as set forth in claim 6 wherein the tetracalcium phosphate is prepared and maintained anhydrously.

10. The method as set forth in claim 1 wherein the dicalcium phosphate is decalcium phosphate anhydrous.

11. A method of producing a powdered calcium phosphate precursor for mixing with an aqueous solution to form a calcium phosphate cement comprising:
wet processing a first calcium phosphate precursor component in an aqueous medium to form a slurry;
freeze drying said first precursor component slurry after said wet processing to form a dry powder; and
mixing said dry first component powder with tetracalcium phosphate powder to form the powdered calcium phosphate precursor.

12. The method as set forth in claim 11 wherein said wet method is a wet milling method.

13. The method as set forth in claim 10 wherein the dicalcium phosphate has a particle size of 1 to 2 µm after milling.

14. The method as set forth in claim 11 wherein the second powdered component is tetracalcium phosphate.

15. The method as set forth in claim 14 wherein the tetracalcium phosphate is made with a calcium to phosphorous ratio of less than 2.

16. The method as set forth in claim 15 wherein the tetracalcium phosphate powder is prepared and maintained anhydrously.

17. The method as set forth in claim 11 further including the step of mixing the powdered tetracalcium phosphate and dicalcium phosphate powder with an aqueous solution to form a setting cement.

18. The method as set forth in claim 12 wherein said wet milling is performed by a bead mill.
